# EUROPEAN PATENT APPLICATION

(11) **EP 3 892 804 A1**
(43) Date of publication of application: **13.10.2021**
(21) Application number: 20382280.4
(22) Date of filing: 07.04.2020
(51) Int. Cl.: E05B 1/00, A61L 2/18

(54) **DISINFECTANT DOSER ATTACHABLE TO A DOOR FOR HANDLE DISINFECTION**

(71) Applicant: Barcelona Technical Center, SL, 08760 Martorell (ES)
(72) Inventor: RUIZ RINCÓN, Rafael, 08760 Martorell (ES); FERREIRO VARELA, Gonzalo, 08760 Martorell (ES); SOTO ESPIGARES, Alberto, 08760 Martorell (ES); VALVERDE CASTAÑO, Mario, 08760 Martorell (ES)
(74) Representative: Juncosa Miró, Jaime

(57) **Abstract**

The invention relates to a door couplable disinfectant dispenser for handles disinfection, comprising, in a first casing (1), a first handle (M1), an initiator member (10) connected to a handle shaft (E), a retarding device (20) connected to the initiator member (10) and actuating a disinfectant triggering device (30) with a certain delay time after the movement of the initiator member (10), and a disinfectant applicator device (40) with at least one first disinfectant emitter (41), fed from a feeder (43), aimed at a first area to be disinfected (A1) over the surface of the first handle (M1), with the degree of dispersion of the disinfectant and the separation existing with the first area to be disinfected (A1) being configured for guaranteeing an approximately uniform and sufficient concentration of disinfectant. In an alternative embodiment, the metering device does not include the first handle, which may be the actual handle of the door.

## Description

### Technical Field

The present invention relates to a door couplable disinfectant dispenser for handles disinfection. Said metering device is configured in a preferred embodiment for applying a disinfectant product directly on the handle after each time the door is opened, ensuring proper disinfection and preventing transmission between users touching said handle.

Handle shall be understood in this description as the mechanism used for opening a door, whether it is a rotating mechanism, a push or pull mechanism, and a lever or knob type mechanism.

The mentioned disinfectant product can be an antiseptic solution of the type normally used for this purpose, but also any disinfecting means, such as a light radiation, susceptible to being supplied in a controlled manner.

According to one embodiment, the metering device integrates a tank with a disinfectant product which is applied on the handles through spray heads.

The metering device of the invention is furthermore equipped with means indicating the aseptic state of the handle, such that a user may know at all times that the metering device has acted correctly.

### State of the Art

Disinfectant dispensers couplable to a door with handles for the disinfection of said handles when the door is opened or closed are known, for example, by means of documents US4046508A and US7080427B1. However, these documents are only applicable to a circular handle and leave only part of said handle accessible, as the rest is introduced inside a disinfection chamber. The outer part of said disinfection chamber is not sterilized and is as accessible for the user as the handle, so the risk of transmission is not prevented with these solutions.

Document US7320418B2 is also known, but this document only proposes sterilizing the handle from one of its sides, leaving the opposite side unsterilized. Furthermore, the solution proposed by this document is actuated only when the door is opened, but not when the handle is actuated. When a door is closed, for example, in a restroom, a user can actuate the handle without opening the door. The solution proposed in this document would not cause the handle to be disinfected in this case. Furthermore, the solution proposed in this document does not allow disinfection of the two handles of one and the same door. This document also requires electric batteries and being actuated by means of an electric motor, which has a high energy consumption and makes the product more expensive, while furthermore making it susceptible to failures.

Another solution is described in document US 6029600, which describes a device envisaged for dispensing for a public restroom user a non-toxic die directly into the hands of a user which is used as a warning and reminder for the user to wash his or her hands. This solution presents the drawback of requiring the collaboration of users.

Documents US7175807B1 and US9649398B1 describe the sterilization of a handle using germicidal ultraviolet light. The second of these documents includes proximity or movement detectors for activating a source of ultraviolet light that is radiated towards the handle, guaranteeing sterilization. However, these solutions involve certain complexity (a computer or control unit, detectors, a UV generator, electric power supply, or a battery power supply) and the manufacture and maintenance cost which impede the solution from being able to be generally applied to any type of doors at an affordable cost.

These and other problems are solved to a great extent by the present invention.

### Brief Description of the Invention

The present invention relates to a door couplable disinfectant dispenser for disinfecting its handles, M1 and M2, envisaged for being installed in doors with any class or type of handles, being configured for being adapted to said doors and to the original handles, or alternatively and preferably being able to replace the original handles.

The invention is particularly applicable in public establishments such as hospitals, restaurants, shopping malls, public baths and generally in any type of areas in which a large number of people must use doors to access certain facilities.

The proposed metering device comprises, in a first casing envisaged for being fixed on a first side of a door, and in a known manner in the state of the known art, as referred to above, the following elements:
- an initiator member movable by means of its interaction with parts of the door during the opening or closing thereof;
- a disinfectant applicator device including at least one first disinfectant emitter susceptible to being arranged adjacent to and aimed at a first handle and fed from a feeder (in general a tank), and
- a triggering device which, activated by the initiator member, actuates the applicator device;

It will therefore be understood that when the initiator member is installed, it is in contact with parts of a door and is configured for experiencing a movement every time a user makes use of said door, i.e., upon opening and/or closing it by acting on at least said first handle

The initiator member is connected to a triggering device such that the movement of the initiator member causes the actuation of said triggering device.

When the triggering device is activated, it actuates the disinfectant applicator device which is in charge of applying disinfectant at least on one first handle of the door through at least one first disinfectant emitter.

Therefore, once the metering device described up until now has been installed on a door, every time the door is opened and/or closed, by acting on one of its handles M1 or M2, it causes the movement of the initiator member which actuates the triggering device, which in turn activates the disinfectant applicator device, causing the disinfection of a first handle of the door opposite said at least one disinfectant emitter.

However, the present invention furthermore proposes the following features, which are not known in the existing state of the art:
- the initiator member is operatively connected to a shaft E which attaches the first handle to the door, and in one embodiment the initiator member includes a hole configured for being coaxially coupled inserted around a handle shaft E attaching the first handle M1 to the door, whereby said initiator member is rotational, rotating in a vertical plane, around said handle shaft E, when the handle M1 is actuated and being interposed between the first handle M1 and a first side of the door;
- said first casing forms an assembly with the first handle, i.e., it is integral therewith, which allows replacing a standard handle with this assembly, with said at least one first disinfectant emitter being aimed at a first area to be disinfected defined on the surface of the first handle, with the degree of dispersion of the disinfectant emitted by said at least one first disinfectant emitter and the separation existing between said at least one first disinfectant emitter and the first area to be disinfected being configured for guaranteeing a sufficient and approximately uniform concentration of disinfectant over the entire surface of the first area to be disinfected; and
- a retarding device (20) is connected to and activated by the initiator member (10) and is interposed between the initiator member (10) and the triggering device (30), with said retarding device being configured for actuating the triggering device (30) with a certain delay time after the rotational movement of the initiator member (10).

That is, after the actuation of the initiator member, application of the disinfectant does not occur until a certain delay time later, giving the user time to let go of the handle and thereby ensuring that disinfection occurs on the handle and not on the user's hand. Preferably said certain delay time can be set.

To protect the two handles M1 and M2 of a door, the mentioned applicator device further includes a second casing equipped with a centering hole envisaged for being arranged inserted around the handle shaft (E) with the second casing being envisaged for being fixed on a second side of said door, the centering hole being aligned with the hole of the initiator member and said second casing including a second handle M2 and at least one second disinfectant emitter 42 aimed at a second area to be disinfected defined on the surface of said second handle M2, with the degree of dispersion of the disinfectant emitted by said at least one second disinfectant emitter and the separation existing between said at least one second disinfectant emitter and the second area to be disinfected being configured for guaranteeing an approximately uniform concentration of disinfectant over the entire surface of the second area to be disinfected and sufficient for guaranteeing the correct disinfection of said area to be disinfected with a single dose of disinfectant applied by the disinfectant emitters.

The proposed disinfectant dispenser includes a hollow handle shaft connecting the first handle M1 and the second handle M2, and the feeder in this preferred embodiment is integrated inside said first casing (1) surrounding the handle shaft E completely (for example, with a toroidal configuration) or partially (for example, with a U-shaped configuration, making it easier to remove it from the tank).

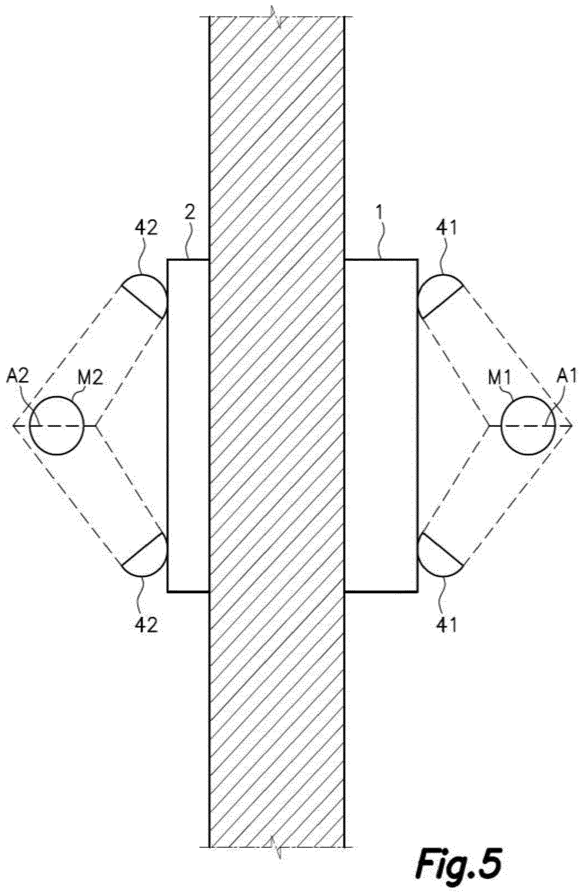

The proposed assembly will include at least two first disinfectant emitters facing opposite sides of one and the same first handle M1 or of the first area to be disinfected A1 and/or said at least one second disinfectant emitter comprises at least two second disinfectant emitters facing opposite sides of the same second handle M2 or of the second area to be disinfected A2, as shown in Figure 5.

The invention in this embodiment provides a first metering device and handle assembly, including a feeder of the disinfectant product (generally a tank) susceptible to replacing a first handle M1, and second handle assembly associated with a casing housing a disinfectant applicator device, suitable for replacing a second handle.

In this preferred embodiment, it has further been envisaged to include in said first handle and metering device assembly or handle and casing assembly with a sprayer a warning assembly implemented by means of a light indicator (for example, a LED, with the capacity for multi-colored signalling), a visual indicator (a signalling unit moved to a warning position by the mentioned initiator element in each actuation), or a sound indicator informing about the aseptic state of the handle or its non-aseptic situation. The warning assembly could also be mechanical, such that the actuation of the initiator member upon interaction with the triggering device will move said signalling unit by way of an indicator flag protruding through a hole of the first and second casing or that is visible through a hole or a transparent part of said first and/or second casing.

Preferably said indicator would include at least one disinfected handle signal and one non-disinfected handle signal. The indicator would change from the disinfected handle position to the non-disinfected handle position when the rotation of the initiator member occurs and would not change back to the disinfected handle position until the actuation of the triggering device.

For example, it is proposed for said indicator to be linked to the plunger of the retarding device, and, provided that said plunger is not at the second end, to indicate that the handle is not disinfected, and only when the plunger is at the second end, to indicate that the handle is disinfected.

The indicator device could also be linked to the triggering device or to the tank so that, when there is no disinfectant liquid left in said tank, the indicator will remain in the non-disinfected handle position even when the plunger is located at the second end of the chamber, or the indicator will be situated in a refill needed position.

By applying solutions from the state of the art, proximity detectors could additionally be applied in combination with the described solution.

In a second embodiment, a solution is proposed wherein the preexisting handles in a door are utilized, and only the metering device is installed attached to the door, which can be coupled to said handles, in the proximity thereof.

The usual handles to which this second embodiment is applied extend horizontally in a standby situation in a plane coinciding with the handle shaft, said handles being separated a certain distance from the door so as to leave space for the user's hand.

In this second embodiment, the initiator member and the retarding member are like those explained above and the mentioned warning assembly can also be implemented, but said at least one first disinfectant emitter is aimed at a first area to be disinfected adjacent to the hole with the first area to be disinfected being spaced apart with respect to the hole by a handle separation in a direction perpendicular to said hole, with the degree of dispersion of the disinfectant emitted by said at least one first disinfectant emitter and the separation existing between said at least one first disinfectant emitter and the first area to be disinfected being configured for guaranteeing an approximately uniform concentration of disinfectant over the entire surface of the first area to be disinfected and sufficient for guaranteeing the correct disinfection of the area to be disinfected with a single application of disinfectant.

Two casings like those explained are likewise used in this case, but they are not attached to a handle, but rather can be coupled to the handle shaft supporting them.

Both the first and the second casings are perfectly positioned with respect to the first handle M1 (coupled in the initiator member) and in the second handle M2 by means of a centering hole once installed on a door, which thereby allows ensuring correct positioning of second disinfectant emitters with respect to the second handle.

The present invention in either of its two embodiments and variants thereof first propose for the initiator member to be able to rotate around a hole envisaged in said initiator member, between a standby position and an actuating position. The mentioned hole of the initiator member is envisaged for being tightly inserted on a handle shaft connecting the first handle with the door, such that the rotational movement of said handle shaft is transmitted to the initiator member.

Said movement will occur when the metering device is attached to a door, in a vertical plane perpendicular to said handle shaft.

Handle shafts typically have a polygonal, preferably square section; therefore, the mentioned hole of the initiator member is proposed to also be preferably polygonal and ideally square.

It is also contemplated that the hole of the initiator member is defined between two portions thereof (taking on the form of a lever) which are fixed to one another at a distance that can be regulated by means of distance regulators, which will normally be screws, which allows moving the two portions closer to one another until the two halves of the hole contact the handle shaft, allowing said handle shaft to be gripped between the two portions of the initiator member.

Therefore, the proposed initiator member causes it to be actuated every time a user causes the handle shaft to rotate by actuating one of the handles of the door.

It is proposed furthermore for the first disinfectant emitters facing a handle in the form of a horizontal lever to be facing a horizontal plane passing through the mentioned hole of the initiator member, specifically focused towards a first disinfection area defined in a portion of said horizontal plane which is adjacent to said hole of the initiator member and which is separated a distance from the handle with respect to the hole of the initiator member, in the direction perpendicular to the hole of the initiator member, and therefore in the direction of the handle shaft when the metering device is in its assembled position on the door.

Given that the first disinfectant emitters are integrated in the first casing together with the initiator member, said first disinfectant emitters are also perfectly positioned in relation to the handle shaft, and therefore with respect to the corresponding first handle attached thereto, when the metering device is installed.

To ensure correct disinfection and maximum utilization of the disinfectant, it is essential for the first disinfectant emitters to be correctly positioned with respect to the first handle and for the degree of dispersion of the disinfectant and the distance between said first disinfectant emitters and the first handle to be correctly set.

Given that the proposed disinfectant dispenser is envisaged for being coupled to any door with handles, the shape, size, and precise position of said handles are unknown, and so as not to require setting actuations during installation, which may not be correctly performed, it is proposed for said first disinfectant emitters, since they are integrated in the first casing together with the initiator member, to be arranged at the time of their manufacture at a predetermined precise distance for generating optimal disinfection on a first area to be disinfected defined on a plane coinciding with the hole of the initiator member and horizontal with the metering device in the installation position.

Therefore, according to this second embodiment, the metering device disinfects both the first handle and the second handle of one and the same door, located on opposite sides thereof, every time one of said first handle or second handle is actuated, but without requiring the components of the metering device to be duplicated, since both the first and the second disinfectant emitters will be connected to the same feeder and actuated by the same triggering device.

For example, it is proposed for the disinfectant dispenser to include a hollow handle shaft, which can replace the handle shaft of any door on which the metering device is installed. In such case, the feeder will be located on the same side of the door as the first disinfectant emitters and the second disinfectant emitters will be connected to said feeder through said handle shaft which is hollow and goes through the door from one side to the other side connecting the first handle to the second handle.

In either of the two described embodiments the mentioned at least two disinfectant emitters will preferably be located one above and the other below the hole of the initiator member, with the metering device being in the installation position, i.e., in the position in which it would be left once it is fixed on a door.

It is likewise proposed for said at least one second disinfectant emitter to be at least two second disinfectant emitters facing opposite sides of the second area to be disinfected.

Preferably, the following features will also be fulfilled:
- the degree of dispersion of each first and/or second disinfectant emitter will be configured for emitting at least 80% of the disinfectant within the first area to be disinfected and/or within the second area to be disinfected, thereby preventing the disinfectant from being misused outside of the first or second area to be disinfected, where the disinfectant is wasted and where the presence of disinfectant may cause problems, such as discoloring, for example, in regions other than the handles due to the repeated application of disinfectant on surfaces not resistant to said disinfectant; and/or
- the first area to be disinfected and/or the second area to be disinfected will have a size comprised between 8 cm and 18 cm long and between 2 cm and 5 cm wide, corresponding to the usual range of sizes for most handles; and/or
- the distance from the handle will be comprised between 2 cm and 6 cm, corresponding with the usual range of distances existing between a handle and a door, where the application of disinfectant is not required as it is highly unlikely that there will be a handle.

The present invention proposes two preferred modes of disinfection.

According to the first mode, disinfection occurs by means of a disinfectant product. According to this solution, the feeder will be a tank (refillable or interchangeable) containing the disinfectant product and the disinfectant emitters will be spray heads connected to said tank by means of conduits. In this embodiment, when there are first and second disinfectant emitters on opposite sides of the same door, the mentioned conduit may go through the door through the handle shaft which will be hollow.

In the installation position, the metering device will be interposed between the first handle and one side of the door. When the first handle includes a decorative trim or plate at its base, surrounding the handle shaft, said decorative trim will be fixed on the first casing which will preferably be planar in the region surrounding the hole of the initiator member and will include configurations that allow fixing said decorative trim, such as through holes for installing screws from the decorative trim to the door, or materials suitable for fixing screws on said first casing, for example.

The mechanism of the proposed metering device is designed for being extremely planar, preferably with a total thickness of less than the 20 mm, so as to have a minimum effect on an existing door to which it is envisaged to be applied, generally replacing the original handle. In the first embodiment, the first casing housing the entire metering device will have a larger volume.

The mentioned first casing for any one of the described embodiments has two main planar faces parallel to one another, without any projection protruding from said main planar faces, allowing the indiscriminate placement of any of said planar faces against a side of a door, such that said first casing can be applied both to right-handed handles and to left-handed handles, i.e., to handles extending horizontally to the right or to the left with respect to the handle shaft.

In the second embodiment, the feeder is outside of said first casing. In such case, may be attached to the first casing or located far away from same, connected thereto through a conduit or an electrical cable.

For example, it is contemplated for several metering devices to be fed from a common feeder, for example, from a centralized disinfectant liquid tank, thereby simplifying tank refilling tasks, or from a centralized battery, simplifying the battery replacement or charging actuations.

Said feeder can optionally be outside of said first casing and be removably attached thereto through an anchoring port.

When the feeder is a tank, this tank can be connected to the first casing through said anchoring port, with said tank hanging from the first casing and allowing it to be easily replaced or refilled when it is empty. It is also contemplated for said tank to be transparent or partially transparent, making its level of disinfectant liquid evident.

Said first casing may optionally include two anchoring ports located at a lower end and at an upper end of the first casing, in the installation position, allowing the indiscriminate placement of the first casing face up or face down, but allowing the placement of the tank to always be in the same position, such that said first casing can be applied both to right-handed handles and to left-handed handles. In this embodiment, it is preferable for said first casing to be symmetrical with respect to the hole of the initiator member.

Ideally, the first casing or also the second casing if there is one, will be configured so as not to occupy, in the installation position, a keyhole region located below, adjacent to and vertically aligned with the handle shaft. The installation of the disinfectant dispenser will therefore not interfere with locks or other mechanisms in doors that are normally located right below where the handle shaft is located.

It is also proposed for the retarding device to be a mechanical device, i.e., a device that stores kinetic energy obtained from the actuation of the first handle and releases it with a certain lag time by means of a mechanism fed by said kinetic energy.

In the present embodiment, the mechanical retarding device includes a spring or piston which is elastically deformed when the initiator member activates the retarding device and includes a retarding mechanism retaining the at least partially deformed spring or piston during said certain delay time, releasing it thereafter and causing the activation of the disinfectant applicator device. That is, the initiator member causes the elastic deformation of the spring or of the piston, whether the compression or the expansion, or even the twisting or bending thereof, but when said initiator member releases the retarding device, said retarding device does not immediately return to its initial position releasing the energy stored in the spring or in the piston, but rather a retaining mechanism postpones said return during the mentioned certain lag time.

According to one embodiment, the retarding mechanism is a plunger which can move in a tight-fitting manner throughout a chamber equipped with a one-way flow control valve at one end and of a quick release hole in an intermediate region. The one-way flow control valve allows the plunger to move freely in one direction when the plunger tries to move in the opposite direction driven by the spring, or the piston only allows a limited air input into the chamber in which the plunger moves, delaying the movement thereof. When said plunger surpasses said release hole of the intermediate region, air freely enters the chamber, which allows the rapid movement of the plunger which, at the end of its stroke, hits the triggering device, activating it.

When the disinfectant is a liquid, it is proposed, for example, for the tank to contain a pressurized gas and for the triggering device to include a valve that can be activated by the retarding device and interposed in the conduit connecting the tank with the spray heads. The valve must allow being activated in an intermediate position of the retarding device, being blocked again at the end of travel of the retarding device in only one direction because if it does this during the activation travel of the retarding device, it will spray the user's hand. For it to actuate in only one direction, a spring- or gravity-activated retractable pawl may be implemented.

There are also contemplated other embodiments, such as the initiator member also actuating a pressurizing device which introduces pressurized gas into the tank every time it is actuated, for example.

There are also contemplated other embodiments according to which the retarding device and/or the triggering device are electronic devices, also fed from a power supply or from an electric battery which can be said feeder of the disinfectant emitters or others. In this case, if the disinfectant is a liquid the valve interposed between the tank and the spray heads will be an electrically operated valve controlled by said triggering device.

All the other features described above in reference to the first embodiment of the disinfectant dispenser which does not integrate handles can likewise be implemented in this second embodiment of the disinfectant dispenser which does integrate handles.

Other features of the invention will become apparent in the following detailed description of an embodiment.

### Brief Description of the Figures

The preceding and other advantages and features will be better understood based on the following detailed description of an embodiment in reference to the attached drawings, which are to be interpreted in non-limiting and illustrative manner, wherein:

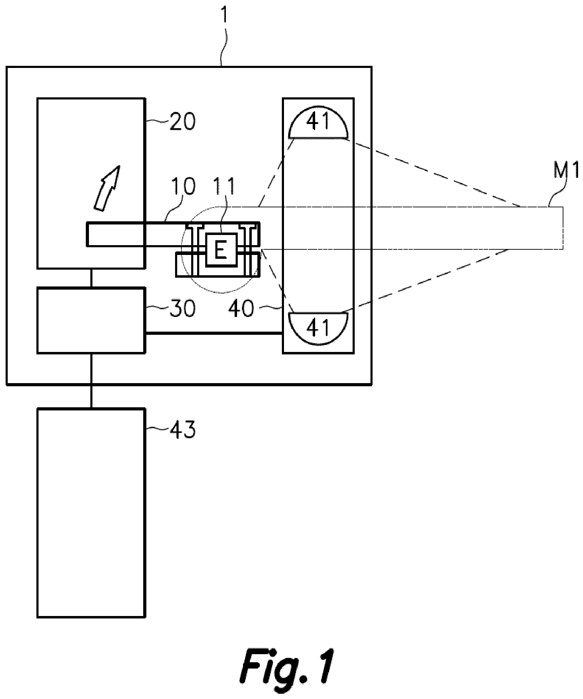

Figure 1 shows a front elevational schematic view of the proposed disinfectant dispenser, shown coupled to a handle shaft of a first handle, and wherein the application cone of the disinfectant on the first handle is indicated with discontinuous lines;

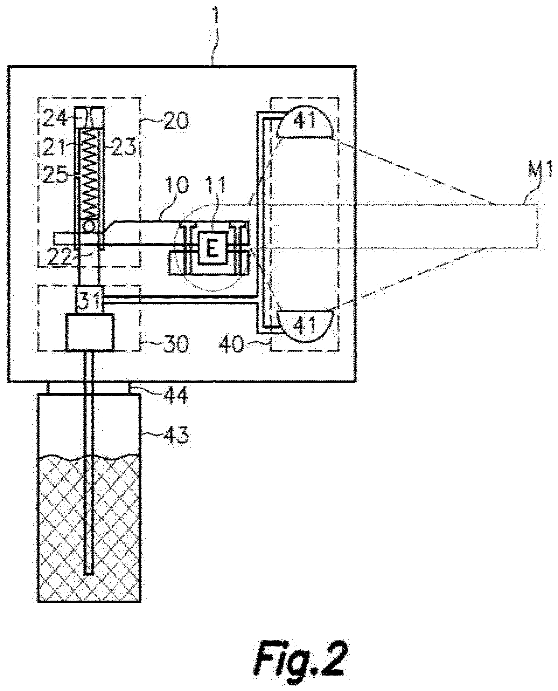

Figure 2 shows the same as Figure 1 but according to a preferred embodiment in which the first disinfectant emitters are spray heads and the feeder is a disinfectant liquid tank connected to said spray heads by means of conduits, and wherein one embodiment of the retarding device is shown in detail;

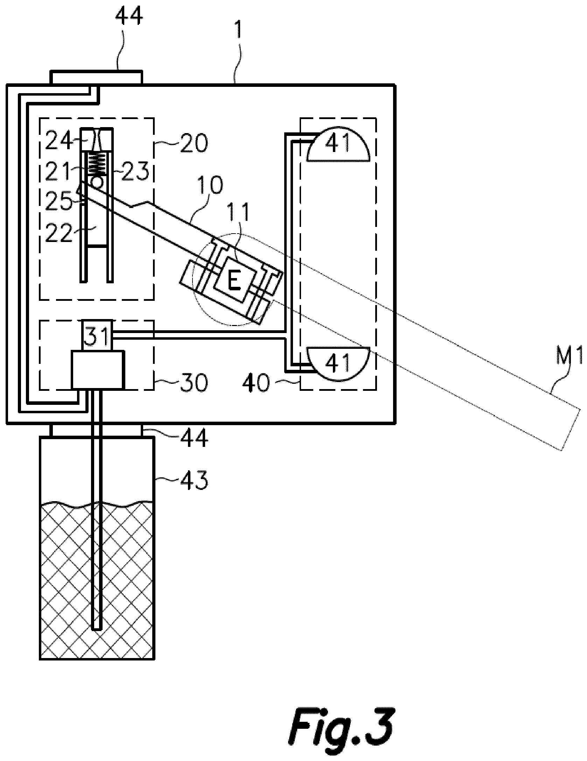

Figure 3 shows the same as Figure 2 but after the actuation of the first handle, showing how the initiator member causes compression of the spring contained in the retarding device which, when the first handle returns to the standby position, will expand again, returning the plunger to the position shown in Figure 2, but with a certain delay time, and this Figure 3 furthermore includes a second docking port on an opposite side of the first casing;

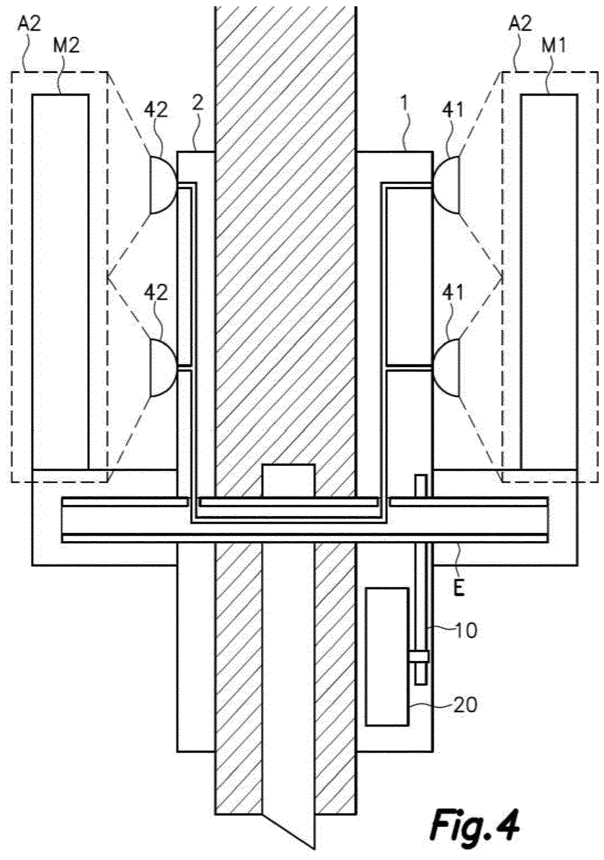

Figure 4 shows a horizontal section of the door taken along the handle shaft, with a first casing attached to one side and a second casing attached to the other side of the door, each casing including several disinfectant emitters facing one and the same side of one and the same area to be disinfected so as to cover in a combined manner the entire surface thereof, said first and second area to be disinfected being shown as a discontinuous line rectangle in this figure;
Figure 5 shows a vertical section of the door taken along the first and second handles, according to one embodiment in which each casing includes several disinfectant emitters, some located above and others below said horizontal plane containing the area to be disinfected and all of them facing said area to be disinfected so as to achieve their disinfection both above and below;

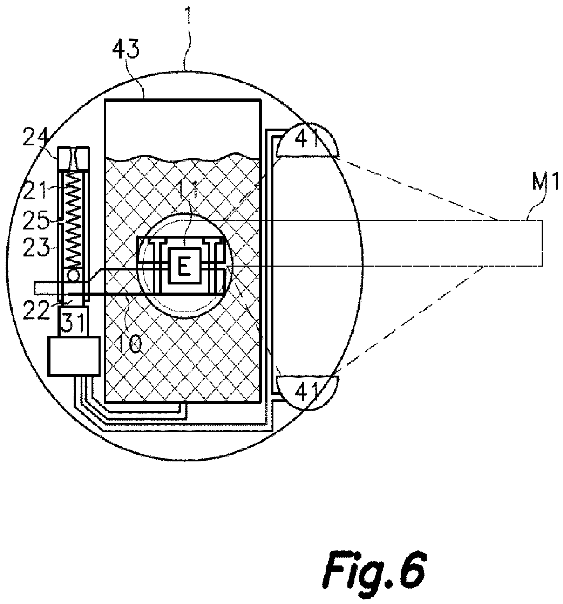

Figure 6 shows an alternative embodiment of the disinfectant dispenser according to which the first casing is circular and concentric to the handle shaft, occupying the place of the shield of the handle, and contains a disinfectant tank completely surrounding said handle shaft;

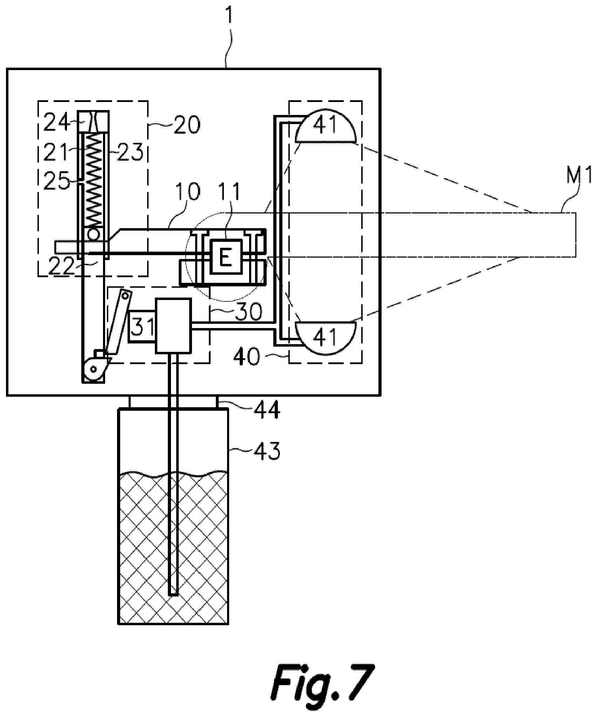

Figure 7 shows an embodiment similar to the one shown in Figure 2 but in which the plunger of the retarding device includes, at a protruding end of the chamber, a one-way switch which, when the plunger is moved towards the second end, presses the valve of the triggering device directly or, as in this example, through an interposed lever, said one-way switch releasing the valve when the plunger reaches the second end. When the plunger is moved in the opposite direction, actuated by the initiator member, the one-way switch is withdrawn without actuating the valve, to subsequently return to its initial position.

### Detailed Description of an Embodiment

The attached figures show non-limiting illustrative embodiments of the present invention.

The proposed disinfectant dispenser is envisaged for being able to be coupled to a door equipped with a first handle M1 connected to said door through a handle shaft E, with the disinfectant dispenser being automatically activated after the opening or closing of the door by actuating said handle, by applying disinfectant on the mentioned first handle M1.

In the embodiment of Figure 1, the proposed disinfectant dispenser consists of a first casing 1 fixed on the surface of the door, around the handle shaft E, and is configured for applying a disinfectant liquid, for example, on a first area to be disinfected A1 defined on the first handle M1 by means of a disinfectant applicator device 40 including first disinfectant emitters 41 which are connected to a feeder 43. The position of said first disinfectant emitters 41 in the first casing 1 is envisaged so that when the first casing 1 is installed on a door, around the handle shaft E, said first disinfectant emitters 41 are adjacent to and facing the first area to be disinfected A1 of the first handle M1.

Typically, the first handle M1 extends horizontally from the end of the handle shaft E to the right or to the left, according to the type of door. Therefore, the first disinfectant emitters 41 will be configured for emitting disinfectant on a horizontal plane coinciding with the handle shaft M1 containing said first area to be disinfected A1.

In the preferred embodiment, the first disinfectant emitters 41 are two first disinfectant emitters 41 located one above and the other below said horizontal plane and are both facing said horizontal plane, such that the upper and lower parts of the handle, as well as its sides are coated with disinfectant.

It is also contemplated for several first disinfectant emitters 41 to be facing the same side of the first handle M1, as shown in Figure 4, facilitating a more uniform emission of disinfectant over its surface.

According to a preferred embodiment of the invention shown in Figure 2, the disinfectant emitters 41 are disinfectant liquid spray heads and are connected through conduits with the feeder 43 which in this embodiment is a tank for holding a disinfectant liquid, such as a hydroalcoholic solution, which is located outside of the first casing 1 but removably attached thereto through a docking port 44. Releasable locking means, such as a key or a numerical combination, can be provided to regulate access to said disinfectant liquid.

In any case, to activate the applicator device 40, the proposed disinfectant dispenser will furthermore include an initiator member 10, which will be moved by the actuation of the first handle M1, a triggering device 30 in charge of activating the applicator device 40, and a retarding device 20 interposed between the initiator member 10 and the triggering device 30 for introducing a certain delay time between the activation of initiator member 10 and the activation of the triggering device 30, giving the user time to release the first handle M1. This mechanism is schematically illustrated in Figure 1.

The initiator member in this embodiment is equipped with a hole 11, in this case a square hole, which is arranged inserted around said handle shaft E, which is also square, such that the rotation of said handle shaft E causes the rotation of the initiator member 10. Said initiator member 10 has two portions with a separation that can be regulated, with the hole 11 being defined between them. This allows the initiator member 10 to act as a clamp tightening around the handle shaft E, achieving the firm attachment thereof.

Therefore, the rotation of the first handle M1 will cause the rotation of the initiator member 10, which actuates the retarding device 20.

In this case, the initiator member 10 has one end, away from the hole 11, which interacts with the retarding device 20, actuating it with its movement.

According to a preferred embodiment of the retarding device 20 illustrated in Figures 2 and 3, such device consists of an elongated chamber 23 inside of which a plunger 22 travels in a tight manner. The chamber 23 also includes a one-way flow control valve 24 at a first end, a quick release hole 25 in a central portion, an O-ring between them which ensures the leak-tightness between the plunger 22 and the chamber 23, an opening at a second end, and a spring 21 contained between the plunger 22 and the first end pushing the plunger 22 towards the second end, where part of the plunger 22 protrudes from the chamber 23 and presses a valve 31 actuating the triggering device 30.

There is also contemplated an embodiment according to which the plunger actuates the triggering device 30 before reaching the second end of the chamber 23, releasing the triggering device 30 upon reaching the second end and therefore preventing the triggering device 30 from remaining activated by the plunger 22. The part of the plunger that presses the triggering device 30, or an interposed mechanism, can be one-way, i.e., it only activates the triggering device 30 when the plunger 22 is moved from the first end to the second end, but not when it is moved in the opposite direction, thereby preventing disinfectant from being discharged onto the user's hand when the first handle M1 is moved, loading the retarding device 20. This can be achieved, for example, by means of a retractable pawl or a catch mechanism.

When the first handle M1 is actuated by a user, the rotation of the initiator member 10 about a vertical plane perpendicular to the handle shaft E occurs. The end of the initiator member 10 is kinematically connected with the plunger 22 and pushes it towards the first end of the chamber 23, compressing the spring 21, as shown in Figure 3. The one-way flow control valve 24 is configured for allowing the unrestricted output of the air contained inside the chamber 23 during this movement.

When the first handle M1 returns to its initial position, the plunger 22 is released from the initiator member 10, and therefore the spring 21 pushes said plunger towards the second end of the chamber 23, but the one-way flow control valve 24 is configured for only allowing a limited air input, which can be regulated, into the chamber 23 during this movement, slowing it down during a certain delay time. When the plunger 21 surpasses the quick release hole 25, air can freely enter the chamber 23 through same, and the spring then rapidly pushes the plunger 21 towards the second end of the chamber 23. When the plunger 21 reaches the end of its stroke, it hits against the valve 31 of the triggering device 30, causing activation of the applicator device 40.

This allows the user to have time to release the first handle M1 after actuating it, ensuring that the disinfectant is emitted on the first handle M1.

This solution of the retarding device is a simple, reliable, low-maintenance, low-cost, and purely mechanical solution, and furthermore it does not require other supply sources, such as electrical energy from a remote supply or a battery, for example.

Other also purely mechanical embodiments of the retarding device 20 are also contemplated, such as for example the loading of a coil spring which actuates a clock mechanism which retains the release of the coil spring during a certain delay time and then release it.

Electronic solutions for the retarding device 20 are also envisaged, for example, the initiator member 10 can actuate a switch which starts a digital clock which, after the course of certain delay time, sends a signal to the triggering device 30 for the actuation of the applicator device 40. In this embodiment, when the disinfectant is a disinfectant liquid, the triggering device 30 can be an electrically operated valve controlled by said retarding device 20.

There is also contemplated an embodiment of the invention according to which just one disinfectant dispenser can simultaneously disinfect the first handle M1 and a second handle M2 located on opposite sides of one and the same door without duplicating the mechanisms thereof.

According to the embodiment shown in Figure 4, the disinfectant dispenser, in addition to the first casing 1 up until now described with the corresponding initiator member 10, retarding device 20, triggering device 30, and applicator device 40 with first disinfectant emitters 41, will also comprise a second casing 2, having a much smaller dimension or thickness, intended for being fixed on one side of the door opposite the side supporting the first casing 1, also adjacent to the handle shaft E going through the door and connecting the first and second handles M1, M2.

The second casing 2 contains only second disinfectant emitters 42 facing a second area to be disinfected A2 defined on the second handle M2 or on a horizontal plane coinciding with the handle shaft E.

Said second disinfectant emitters 42 are actuated by the same triggering device 30 and connected to the same feeder 43 as the first disinfectant emitters 41. Given that both the triggering device 30 and the feeder 43 are on the side of the door supporting the first casing 1, the passage of connections between the first casing 1 and the second casing 2 is required. For example, if the disinfectant is a liquid, a conduit going through the door from one side to the other side will be required.

This connection can be done through a hole made in the door, but preferably it will be done through the handle shaft E, which is proposed to be hollow, thereby preventing making new holes in the door. Figure 4 shows this embodiment, the feeder 43 although is not observed therein as the section is taken along a plane that does not include said feeder 43.

In one embodiment of the invention, it has been envisaged for the mentioned first casing 1 to internally integrate the entire metering device and the feeder 43 as well, whether it is a disinfectant supply tank or means for providing disinfecting radiation, such that this casing housing the initiator member 10 will be coupled inserted around the handle shaft E, as shown in Figure 6, occupying the base of said handle (a region usually occupied by a shield) and surrounding said handle shaft E. The second casing 2 containing the disinfectant emitters will likewise occupy the base region of the handle, surrounding the handle shaft E. This solution allows utilizing existing handles.

Still in an alternative preferred embodiment, it is proposed for both the first casing and the second casing to integrate a handle, such that an assembly suitable for replacing a preexisting handle, utilizing the handle shaft E if possible, is proposed.

This solution described below provides as an important advantage of said disinfectant emitters, that since they are integrated in the first casing 1 or second casing 2 together with the initiator member 10, they are arranged at the time of their manufacture at a predetermined precise distance for generating optimal disinfection on the area of the handle (which may be of any format: lever, knob, etc.) to be disinfected

It will be understood that the different parts constituting the invention described in one embodiment may be freely combined with the parts described in other different embodiments, even though said combination may not have been explicitly described, provided that such combination is not detrimental.

In particular, it would be possible to implement the invention using as a disinfectant emitter an ultraviolet or other type of light source (such as infrared light in a predetermined wavelength band) using the mentioned previously disclosed teachings on this type of application (triggering device in the form of a switch actuating the ultraviolet light sources, and being wire- or battery-powered).

The operative connection of the initiator member 10 with the shaft E of the first handle M1 could likewise be done through a gear train rather than through an initiator member in the form of a lever with an openworked hole in the mentioned shaft E.

## Claims

1. A door couplable disinfectant dispenser for handles disinfection, comprising, in a first casing (1) envisaged for being fixed on a first side of a door:
• an initiator member (10) movable by means of interaction thereof with parts of the door during the opening or closing thereof;
• a disinfectant applicator device (40) including at least one first disinfectant emitter (41) arrangeable adjacent to and aimed at a first handle (M1) and fed from a feeder (43), and
• a triggering device (30) which, activated by the initiator member (10), actuates the applicator device (40);
**characterized in that**
• the initiator member (10) is operatively connected to a shaft (E) which attaches the first handle (M1) to the door, such that when said first handle is moved produces a corresponding movement of the initiator member (10);
• said first casing (1) integrates the first handle (M1), with said at least one first disinfectant emitter (41) being aimed at a first area to be disinfected (A1) defined on the surface of the first handle (M1), with a degree of dispersion of the disinfectant emitted by said at least one first disinfectant emitter (41) and a separation existing between said at least one first disinfectant emitter (41) and the first area to be disinfected (A1) being configured for guaranteeing an approximately uniform concentration of disinfectant over the entire surface of the first area to be disinfected and sufficient for the disinfection thereof; and **in that**
• a retarding device (20) is connected to and activated by the initiator member (10) and is interposed between the initiator member (10) and the triggering device (30), said retarding device being configured for actuating the triggering device (30) with a certain delay time after the rotational movement of the initiator member (10).

2. The disinfectant dispenser according to claim 1, wherein the initiator member (10) includes a hole (11) configured for being coupled to the handle shaft (E) which attaches the first handle (M1) to the door, the initiator member (10) being rotational in a vertical plane and being interposed between the first handle (M1) and a first side of the door.

3. The disinfectant dispenser according to claim 1 or 2, wherein said applicator device (40) further includes a second casing (2) equipped with a centering hole arranged for being inserted around the handle shaft (E), with the second casing (2) being envisaged for being fixed on a second side of said door, the centering hole being aligned with the hole (11) of the initiator member (10), said second casing (2) including a second handle (M2) and at least one second disinfectant emitter (42) aimed at a second area to be disinfected (A2) defined on the surface of said second handle (M2), with a degree of dispersion of the disinfectant emitted by said at least one second disinfectant emitter (41) and the separation existing between said at least one second disinfectant emitter (41) and the second area to be disinfected (A2) being configured for guaranteeing an approximately uniform concentration of disinfectant over the entire surface of the second area to be disinfected and sufficient for the disinfection thereof.

4. The disinfectant dispenser according to claim 3, wherein the disinfectant dispenser includes a hollow handle shaft (E) connecting the first handle (M1) and the second handle (M2), and wherein the feeder (43) is integrated inside said first casing (1) completely or partially surrounding the handle shaft (E).

5. The disinfectant dispenser according to any one of claims 1 to 4, wherein said at least one first disinfectant emitter (41) are at least two first disinfectant emitters (41) facing opposite sides of the same first handle (M1) and/or said at least one second disinfectant emitter (42) are at least two second disinfectant emitters (42) facing opposite sides of the same second handle (M2).

6. A door couplable disinfectant dispenser for handles disinfection, comprising in a first casing (1) envisaged for being fixed on a first side of a door:
• an initiator member (10) movable by means of the interaction thereof with parts of the door during the opening or closing thereof;
• a disinfectant applicator device (40) including at least one first disinfectant emitter (41) arrangeable adjacent to and aimed at a first handle (M1) which is horizontal in a standby situation, and with said first disinfectant emitter (41) being fed from a feeder (43), and
• a triggering device (30) which, activated by the initiator member (10), actuates the applicator device (40);
**characterized in that**
• the initiator member (10) is operatively connected to a shaft (E) attaching the first handle (M1) to the door, such that when said handle is moved, it brings about a corresponding movement of the initiator member (10);
• said at least one first disinfectant emitter (41) is aimed at a first area to be disinfected adjacent to the hole (11) and defined on a horizontal plane passing through said hole (11), with the first area to be disinfected being spaced apart with respect to the hole (11) by a handle separation in a direction perpendicular to said hole (11), with a degree of dispersion of the disinfectant emitted by said at least one first disinfectant emitter (41) and a separation existing between said at least one first disinfectant emitter (41) and the first area to be disinfected being configured for guaranteeing an approximately uniform concentration of disinfectant over the entire surface of the first area to be disinfected, and sufficient for the disinfection thereof; and **in that**
• a retarding device (20) is connected to and activated by the initiator member (10) and is interposed between the initiator member (10) and the triggering device (30), with said retarding device being configured for actuating the triggering device (30) with a certain delay time after the rotational movement of the initiator member (10).

7. The disinfectant dispenser according to claim 6, wherein the initiator member (10) includes a hole (11) which is configured for being coupled to a handle shaft (E) attaching the first handle (M1) to the door, with the initiator member (10) being rotational in a vertical plane and being interposed between the first handle (M1) and a first side of the door;

8. The disinfectant dispenser according to claim 6, wherein said applicator device (40) further includes a second casing (2) equipped with a centering hole which is arranged for being inserted around the handle shaft (E), holding a second handle (M2), with the second casing (2) being envisaged for being fixed on a second side of said door, the centering hole being aligned with the hole (11) of the initiator member (10), said second casing (2) including at least one second disinfectant emitter (42) aimed at a second area to be disinfected located on an opposite side of the door with respect to the first area to be disinfected, wherein said second area to be disinfected is adjacent to the centering hole and is defined on a horizontal plane passing through said centering hole, with the second area to be disinfected being spaced apart with respect to the centering hole by a handle separation in a direction perpendicular to said centering hole, with the degree of dispersion of the disinfectant emitted by said at least one second disinfectant emitter (41) and the separation existing between said at least one second disinfectant emitter (41) and the second area to be disinfected being configured for guaranteeing an approximately uniform concentration of disinfectant over the entire surface of the second area to be disinfected and sufficient for the disinfection thereof, and with the first and second disinfectant emitters (41, 42) being activated by the same triggering device (30) and fed from the same feeder (43) through the door.

9. The disinfectant dispenser according to claim 7, wherein the disinfectant dispenser includes a hollow handle shaft (E), the feeder (43) is located on said first side of the door, and wherein the second disinfectant emitters (42) are connected to said feeder (43) through said hollow handle shaft (E).

10. The disinfectant dispenser according to claim 6, 7, or 8, wherein said at least one first disinfectant emitter (41) are at least two first disinfectant emitters (41) facing opposite sides of the same first area to be disinfected and/or said at least one second disinfectant emitter (42) are at least two second disinfectant emitters (42) facing opposite sides of the same second area to be disinfected.

11. The disinfectant dispenser according to any one of preceding claims 1 to 5 or 6 to 10, wherein the first casing (1) has two main planar faces parallel to one another, without any projection protruding from said main planar faces, allowing the indiscriminate placement of any of said planar faces against a side of the door, such that said first casing (1) can be applied both to right-handed handles and to left-handed handles.

12. The disinfectant dispenser according to any one of preceding claims 1 to 3, 5, or 6 to 10, wherein the feeder (43) is outside of said first casing (1) and is removably attached thereto through an anchoring port (44), and wherein the first casing (1) includes two anchoring ports (44) located at a lower end and at an upper end of the first casing (1), allowing the indiscriminate placement of the first casing (1) face up or face down, allowing the placement of the feeder (43) in any of said anchoring ports (44), according to whatever the position of the first casing (1) is, such that said first casing (1) can be applied both to right-handed handles and to left-handed handles.

13. The disinfectant dispenser according to any one of preceding claims 1 to 5 or 6 to 11, wherein the retarding device (20) is a mechanical device including a spring (21) or piston which is elastically deformed when the initiator member (10) activates the retarding device (20) and including a retarding mechanism (22, 23, 24, 25) retaining the at least partially deformed spring (21) or piston during said certain delay time, releasing it thereafter and causing the activation of the actuating device (30).

14. The disinfectant dispenser according to claim 13, wherein the retarding mechanism (22, 23, 24, 25) is a plunger (22) which can move in a tight-fitting manner throughout a chamber (23) equipped with a one-way flow control valve (24) at one end and of a quick release hole (25) in an intermediate region.

15. The disinfectant dispenser according to any one of preceding claims 1 to 5 or 6 to 13, wherein the feeder (43) is a disinfectant product tank containing a pressurized gas, in connection with, by means of a conduit, said first and/or second disinfectant emitters (41, 42) which are disinfectant product spray heads, and wherein the triggering device (30) includes a valve (31) that can be activated by the retarding device (20) and is interposed in said conduit.

16. The disinfectant dispenser according to any one of the preceding claims, wherein:
• the degree of dispersion of each first and/or second disinfectant emitter (41, 42) is configured for emitting at least 80% of the disinfectant within the first area to be disinfected and/or within the second area to be disinfected; and/or
• the first area to be disinfected and/or the second area to be disinfected have a size comprised between 8 cm and 18 cm long and between 2 cm and 5 cm wide; and/or
• the distance from the handle is comprised between 2 cm and 6 cm.

17. The disinfectant dispenser according to any one of the preceding claims, wherein it further comprises integrated in said first and second casings (1, 2) a warning assembly implemented by means of a light, visual, or sound indicator informing about the aseptic state of the handle or the non-aseptic situation thereof.
